# EUROPEAN PATENT SPECIFICATION

(11) **EP 3 580 673 B1**
(45) Date of publication and mention of the grant of the patent: **25.02.2026**
(21) Application number: 18701037.6
(22) Date of filing: 22.01.2018
(51) Int. Cl.: G16H 40/67, G16H 20/17, A61M 5/14

(54) **SYSTEM AND METHOD FOR COMMUNICATING INFORMATION BETWEEN A MULTIPLICITY OF MEDICAL PUMP DEVICES AND AT LEAST ONE COMMUNICATION DEVICE**
SYSTEM UND VERFAHREN ZUR KOMMUNIKATION VON INFORMATIONEN ZWISCHEN EINER VIELZAHL VON MEDIZINISCHEN PUMPVORRICHTUNGEN UND MINDESTENS EINER KOMMUNIKATIONSVORRICHTUNG
SYSTÈME ET PROCÉDÉ DE COMMUNICATION D'INFORMATIONS ENTRE UNE MULTIPLICITÉ DE DISPOSITIFS DE POMPE MÉDICALE ET AU MOINS UN DISPOSITIF DE COMMUNICATION

(30) Priority: 08.02.2017 EP 17305146
(43) Date of publication of application: 18.12.2019
(73) Proprietor: Fresenius Vial SAS, 38590 Brézins (FR)
(72) Inventor: MERMET, Emeric, 38950 Saint Martin le Vinoux (FR)
(74) Representative: Maikowski & Ninnemann Patentanwälte Partnerschaft mbB
(86) International application number: PCT/EP2018/051378
(87) International publication number: WO 2018/145880

(56) References cited:
- US-A1- 2012 226 771
- US-A1- 2012 226 771
- US-A1- 2013 191 513
- US-A1- 2013 191 513
- NICOLAS DUCROT ET AL: "LoRa Device Developer Guide. Orange Connected Objects & Partnerships.", 30 April 2016 (2016-04-30), XP055464950, Retrieved from the Internet <URL:https://partner.orange.com/wp-content/uploads/2017/11/LoRa-Device-Developer-Guide-Orange.pdf> [retrieved on 20180405]
- PETAJAJARVI JUHA ET AL: "Evaluation of LoRa LPWAN technology for remote health and wellbeing monitoring", 2016 10TH INTERNATIONAL SYMPOSIUM ON MEDICAL INFORMATION AND COMMUNICATION TECHNOLOGY (ISMICT), IEEE, 20 March 2016 (2016-03-20), pages 1 - 5, XP032916146, [retrieved on 20160623], DOI: 10.1109/ISMICT.2016.7498898
- PATRICIA TRBOVICH, ANTHONY EASTY: "Smart Pump Implementation: A Guide for Healthcare Institutions", 3 August 2012 (2012-08-03), pages 1 - 10, XP093276942, Retrieved from the Internet <URL:https://www.aami.org/docs/default-source/foundation/infusion/2012_si_smart_pump_implementation.pdf?sfvrsn=1a1c6fa9_4> [retrieved on 20250513]
- � LORA ALLIANCE ET AL: "A technical overview of LoRa What is it?", 31 May 2016 (2016-05-31), XP055326494, Retrieved from the Internet <URL:http://www.semtech.com/wireless-rf/iot/LoRaWAN101_final.pdf> [retrieved on 20161206]
- ALO�S AUGUSTIN ET AL: "A Study of LoRa: Long Range & Low Power Networks for the Internet of Things", SENSORS, vol. 16, no. 9, 9 September 2016 (2016-09-09), CH, XP055365265, ISSN: 1424-8220, DOI: 10.3390/s16091466
- "A Study of LoRa: Long Range & Low Power Networks for the Internet of Things", SENSORS, vol. 16, no. 9, 9 September 2016 (2016-09-09), CH, XP055365265, ISSN: 1424-8220, DOI: 10.3390/s16091466
- LORA ALLIANCE ET AL: "A technical overview of LoRa What is it?", 31 May 2016 (2016-05-31), XP055326494, Retrieved from the Internet <URL:http://www.semtech.com/wireless-rf/iot/LoRaWAN101_final.pdf> [retrieved on 20161206]

## Description

The invention relates to a system for communicating information between a multiplicity of medical pump devices and at least one communication device according to the preamble of claim 1 and to a method for communicating information between a multiplicity of medical pump devices and at least one communication device, according to claim 8.

A system of this kind comprises a server to which at least one communication device is connected. At least one gateway, also denoted as base station, is connected to the server via a network connection. The system furthermore comprises at least one medical pump device, such as an infusion device or a pump device for the enteral feeding of a patient.

There is a desire to provide healthcare to a patient at his/her home. This may help to provide for an increased comfort for the patient and allows the patient to live, potentially, a regular life.

Providing care to a patient at his/her home however comes with the challenge that patients may live far away from a healthcare professional, such as a physician or a nurse in a hospital or in private practice. In addition, patients may live far apart from one another, such that the checking of patients by for example a nurse may require large travel distances to visit one patient after the other. For this reason, patients may be required to operate their medical pump devices autonomously. Hence, patients may have to set up and start a delivery operation on their own according to a prescription which they have obtained from a healthcare professional. This however does not alleviate the need for a regular supervision and checking by a healthcare professional, because if a patient does not follow a prescribed treatment closely or if malfunctions during a delivery operation occur without appropriate action, this may lead to severe effects, potentially requiring a re-hospitalization of the patient.

There is a desire to remotely supervise medical pump devices and their operation. There is furthermore a desire to increase efficiency of a healthcare professional to allow the healthcare professional to spend more time on the actual care. There in addition is a desire to monitor medical pumps by the pump manufacturer in order to gather operational data relating for example to the reliability of a pump, relating to user preferences and relating to potential maintenance needs.

Patent application US2013191513A1 discloses a system and a method for electronic patient care, wherein the system may include an infusion pump which communicates with one or more servers via a gateway using low energy Bluetooth. The infusion pump can also be connected to a wearable system monitor.

The scope of the invention is defined by the appended claims 1-8.

It is an object of the invention to provide a system and a method for communicating information between a multiplicity of medical pump devices and at least one communication device which allow for an efficient remote monitoring of the medical pump devices.

This object is achieved by means of a system comprising the features of claim 1. Accordingly, the at least one medical pump device is connected to the at least one gateway via a low power wide area network connection.

Low power wide area network (in short LPWAN or LPN) stands for a class of network protocols for connecting low energy devices (for example driven by a battery) to a network server. The protocol is constituted such that a long-range communication at a low energy consumption of, in the instant case, the medical pump devices can be achieved, the communication being secure according to medical device regulations.

A suitable network architecture of a low power wide area network is for example the LoRaWAN (short for Long-Range Wide Area Network), which is an industry standard defined by the LoRa Alliance and making use of LoRa modulation. LoRaWAN allows for a bidirectional communication and has been developed for the Internet of Things (IoT). LoRaWAN uses frequencies around 868 MHz in Europe and around 915 MHz in the US and allows for a communication in a long range, for example up to 40 km.

In principle, also other communication protocols may be used, such as Symphony Link, LTE-M, NarrowBand-loT (NB-loT), Weightless-N, Weightless-P, Weightless-W, or Wi-Fi HaLow, which also represent low power wide are networks.

It is a desire that medical pump devices, such as pumps for the enteral feeding or infusion pumps to provide intravenous infusions, may run on battery for an extended period of time, for example for (at least) 24 hours. A wireless communication with a server hence should take place at low energy without a large impact on the device's battery. For this reason, the connection between the medical pump device and the server is established via a gateway, also denoted as base station, which communicates with the medical pump device wirelessly making use of a low power wide area network connection. Using LoRaWAN, the energy consumption during an active communication may for example be around 10 mA and in an idle mode about 100 nA.

A low power wide area network connection typically allows for a data communication at a rather low data rate. For example, for a LoRaWAN connection the data rate may lie between 0.3 kbps and 50 kbps. This generally may suffice to communicate information between a medical pump device and the gateway to allow for example for a remote monitoring of the medical pump device.

The at least one medical pump device comprises a first communication module for establishing the low power wide area network connection to the at least one gateway. The module comprises or is connected to an antenna establishing the wireless low power wide area network connection to the gateway.

The at least one medical pump device, in addition, comprises a second communication module for establishing a communication connection according to a different network technology to another device. Via the second communication module a communication different than the low power wide area network connection may be established, such that the medical pump device may communicate in different ways. The communication connection may for example be a low power connection, for example a Bluetooth connection, in particular a Bluetooth Low Energy connection, allowing for a short range connection to a device located in the vicinity of the medical pump device. Bluetooth Low Energy is an industry standard for a short range communication technology having for example a range of about 10 m, at a low energy consumption. Via the communication connection of the second communication module, hence, a direct connection to the medical pump device may be established, independent of the gateway, such that a user may connect for example via a communication device such as a computer device or a mobile communication device, for example a smart phone or a tablet computer, directly to the medical pump device.

In one embodiment, the at least one medical pump device may comprise a third communication module, in particular a triangulation or localization service module, for example a GPS module, for generating localization information. Via the localization module the location of the medical pump device can be determined, such that localization information may be transmitted from the medical pump device via the gateway to the server.

It is to be noted that a third communication module such as a GPS module for determining the location of the medical pump device may be dispensable. Localization information can also be derived for example using localization services of LoRaWAN.

The medical pump device may in particular be located at a remote location from the server and also from a communication device connecting to the server. In particular, a medical pump device may be located at a patient's home, hence remote from a healthcare professional, such as a physician or a nurse in a hospital or in private practice. By connecting to the server, a healthcare professional may establish a connection to one or multiple medical pump devices, such that a monitoring of the medical pump devices and a data transmission between the medical pump devices and the communication device of the healthcare professional becomes possible.

In one embodiment, a user may access the server via a web application using a communication device such as a computer device or a mobile communication device, for example a smart phone or a tablet computer. The server comprises a database in which information relating to one or multiple medical pump devices is stored and which may be accessed via the web application.

For example, the web application may be constituted to provide information relating to delivery operations carried out by the at least one medical pump device. For example, the web application may provide information about infusion operations that have been carried out throughout a defined time period, for example within the last 30 days. The web application may also provide information relating to current, ongoing delivery operations such as an enteral feeding operation or an infusion operation, such that operations may be monitored as they are being carried out. Via the web application hence an analysis of recent delivery operations as well as a monitoring of actual infusion operations may be carried out.

In addition or alternatively, the web application may provide information relating to alarm conditions of a medical pump device. Alarm conditions herein may relate to an ongoing infusion operation, such that a healthcare professional is alarmed if during a delivery operation a malfunction, such as an occluded delivery line, occurs. Also, information with regard to past alarms, for example with regard to all alarms in a specified time period, for example within the last thirty days, may be provided. In addition, technical alarms may be reported, such as technical failure of equipment of a medical pump device.

In addition or alternatively, the web application may be constituted to provide treatment compliance information of delivery operations carried out by the at least one medical pump device. Generally, a patient in homecare is required to set up and start delivery operations autonomously and herein shall follow closely a treatment prescription obtained from a healthcare professional such as a physician or a nurse. Treatment compliance information hence may relate to information about the extent of conformity to the recommendations for the day-to-day treatment by the healthcare professional with respect to the timing, dosage and frequency and thus can be defined as the extent to which a patient acts in accordance with the prescribed interval and dose of a dosing regimen. Via the web application it hence can be monitored whether a patient followed a prescription at all, and to what extent the patient potentially followed the prescription.

In addition or alternatively, the web application may also output patient information, such as information relating to the patient identification, or information relating to the patient's state, such as his/her current weight, body temperature and the like. For this, external devices such as a scale and a temperature sensing device may communicate, for example also using a low power wide area network connection, via a gateway to the server, such that the server may also obtain and store information other than pump information.

In addition or alternatively, the web application may be constituted to provide information relating to maintenance of the at least one medical pump device. Via the web application, a technical service professional may for example monitor when a medical pump should be serviced next. The web application may for example output the remaining days until the next service is to be performed. Also, the web application may output information relating to the operating software running on the medical device and its current version.

In addition or alternatively, the web application may be constituted to provide information relating to pump settings, such as version information, software and/or hardware information, a language setting or the like.

In addition or alternatively, the web application may be constituted to provide information relating to the localization of the at least one medical pump device. Hence, via the web application a multiplicity of medical pump devices may be localized such that a service professional as well as a healthcare professional may easily obtain information regarding the status and current whereabouts of an installed base of medical pump devices.

Via the low power wide area network connection a bidirectional communication may take place. Hence, information may not only be transmitted from one or multiple pump devices towards the server, but data may also be transmitted in the other direction from the server towards one or multiple medical pump devices.

A data transfer from the server to a medical pump device may for example be used to update software of the medical pump device, such that a medical pump device may be kept up-to-date easily with respect to its operating software.

The object is also achieved by a method for communicating information between a multiplicity of medical pump devices and at least one communication device, in which:
- a server communicates with at least one communication device, and
- at least one medical pump device communicates with at least one gateway connected to the server via a network connection.

Herein, the at least one medical pump device communicates with the at least one gateway via a low power wide area network connection.

The advantages and advantageous embodiments described above for the system equally apply also to the method, such that it shall be referred to the above.

The idea underlying the invention shall subsequently be described in more detail with regard to the embodiments shown in the figures. Herein:
- Fig. 1: shows a schematic overview of a system to communicate data between a multiplicity of medical pump devices and a server;
- Fig. 2: shows a schematic view of a medical pump device in connection with a gateway;
- Fig. 3: shows a start view of a web application running on the server;
- Fig. 4: shows a view of the web application;
- Fig. 5: shows another view of the web application;
- Fig. 6: shows another view of the web application;
- Fig. 7: shows yet another view of the web application;
- Fig. 8: shows yet another view of the web application;
- Fig. 9: shows a scenario of a first use case;
- Fig. 10: shows a scenario of a second use case;
- Fig. 11A, 11B: show scenarios of a third use case;
- Fig. 12: shows a scenario of a fourth use case;
- Fig. 13: shows a scenario of a fifth use case; and
- Fig. 14: shows a scenario of a sixth use case.

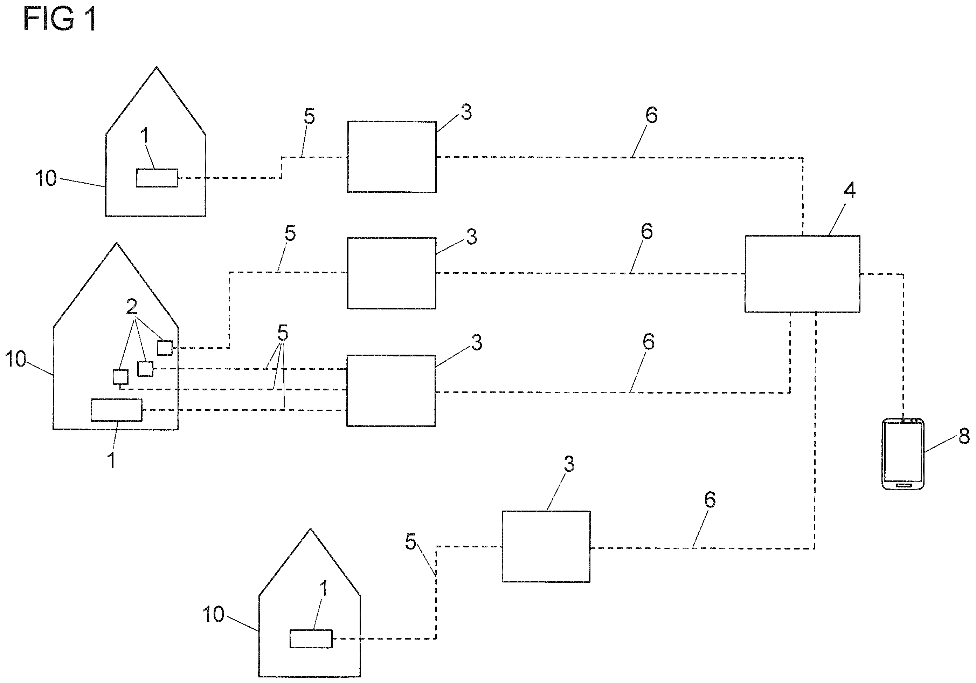

Fig. 1 shows a schematic view of a system for communicating information between medical pump devices 1 and a communication device 8 via a server 4.

The medical pump devices 1 may for example be pumps for the enteral feeding of a patient or infusion pumps for the intravenous infusion of medical fluids such as medication or the like. A medical pump device 1 may for example be located in a patient's home 10 and may serve to provide homecare to a patient. By means of a medical pump device 1 a patient may autonomously set up and carry out delivery operations, hence allowing the patient to treat himself/herself at home without the presence of a healthcare professional such as a physician or a nurse.

The system allows for the remote monitoring of a multiplicity of medical pump devices 1 and allows also for the remote programming and supervision of the medical pump devices 1. Within the system, the medical pump devices 1 are connected to the server 4 via one or multiple gateways 3, which are also denoted as base stations and are constituted to establish a wireless low power wide area network connection 5 to the medical pump devices 1.

In particular, the medical pump devices 1 may communicate with the gateways 3 via a LoRaWAN connection according to the standardized LoRa protocol. LoRaWAN is a low power wide area network (in short LPWAN or LPN) intended for a wireless communication and in particular intended for devices which run of battery and hence should have a low energy consumption. LoRaWAN allows for a bidirectional communication and also offers localization services.

LoRaWAN typically has a star network topology in which the gateways 3 serve as a (transparent) bridge relaying data communication between the medical pump devices 1 and the central server 4. The gateways 3 are connected to the server 4 via standard network connections, in particular via a public communication network such as the Internet providing for IP connections 6 between the gateways 3 and the server 4. LoRaWAN uses data rates in the range from 0.3 kbps to 50 kbps, wherein the data rate may be managed by means of an adaptive data rate (ADR) scheme to maximize battery life of the medical pump devices 1 and the overall network capacity.

The server 4 stores information received from the medical pump devices 1 in a database and may be accessed from communication devices 8 such as computer devices or mobile communication devices (e.g., smart phones or a tablet computers) via a web application. Hence, via the server 4 the medical pump devices 1 may be monitored, localized and serviced, for example by transmitting software updates to the medical pump devices 1.

As schematically indicated in Fig. 1, additional devices 2 such as a scale to measure a patient's weight or a temperature sensing device to measure a patient's body temperature may be connected via low power wide area network connections 5 to gateways 3 and via the gateways 3 to the server 4, such that the server 4 may receive information also from the additional devices 2 and may include the information in its database for access via the web application.

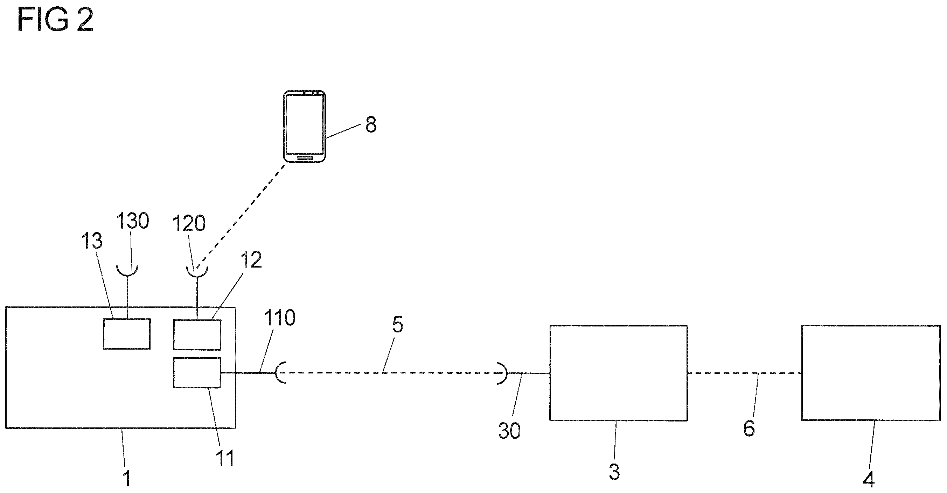

Fig. 2 shows in a schematic drawing a medical pump device 1 in connection with a gateway 3. The medical pump device 1 comprises a first communication module 11 having an antenna 110 for wirelessly communicating with an antenna 30 of the gateway 3. The first communication module 11 is set up to establish a low power wide area network connection 5 to the gateway 3, such as a LoRaWAN connection.

In addition, the medical pump device 1, in the illustrated embodiment, comprises a second communication module 12 having an antenna 120. The second communication module 12 may be constituted to set up a communication connection according to a different network technology to for example a communication device 8, such as a Bluetooth Low Energy connection. In particular, the second communication model 12 may establish a communication connection using a protocol according to a different standard than LoRaWAN. For example, via the second communication module 12 a short range communication connection, such as Bluetooth Low Energy, may be established.

In addition, in the illustrated embodiment the medical pump device 1 comprises a third communication module 13 having an antenna 130. The third communication module 13 may for example be a GPS module and may serve to determine the geographical location of the medical pump device 1.

The GPS module 13 may in principle be dispensable. In particular, LoRaWAN may also offer localization services, such that localization information relating to the medical pump device 1 may also be obtained from LoRaWAN.

The antennas 110, 120, 130 may be individual antennas or may be implemented by a single antenna common to all modules 11, 12, 13.

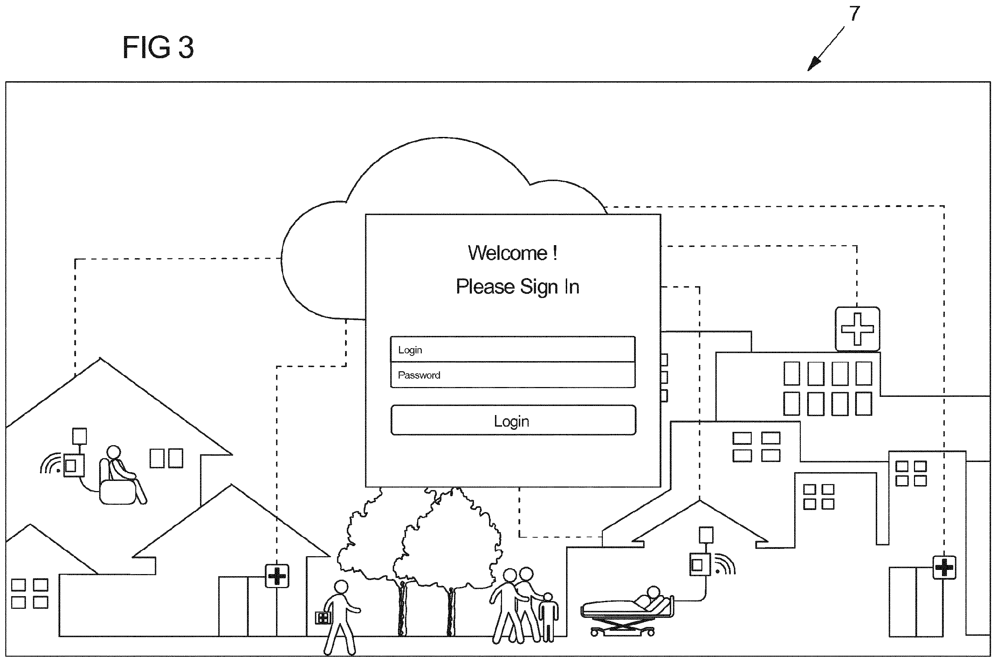

Fig. 3 shows a view of a web application 7 hosted on the server 4 and accessible for example via the Internet using a communication device 8 such as a computer device or a mobile communication device, for example a smart phone or a tablet computer.

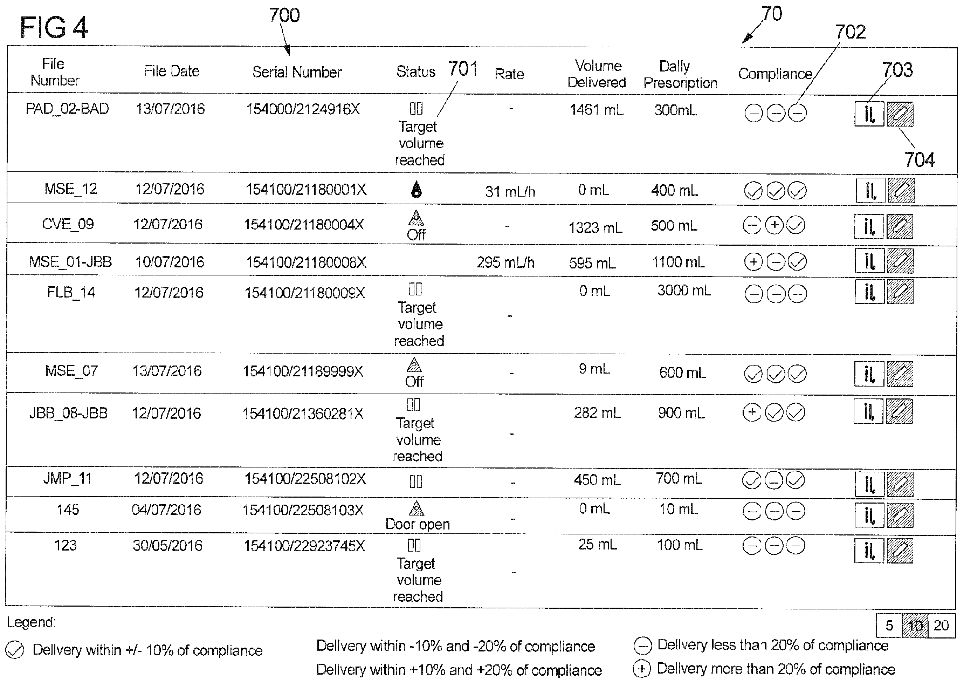

As depicted in Fig. 3, when starting the web application 7 a user is required to authenticate himself/herself by entering a username and a password. Upon successful authentication, a user obtains access to the web application 7 and may, on different views 70 to 74 illustrated in Fig. 4 to 8, perform different functions and obtain different information.

For example, in a first view 70 shown in Fig. 4, the user is displayed with a list of medical pump devices 1 which are associated with the user. For example, a healthcare professional such as a physician or a nurse may have the authorization to gain access to a predefined set of medical pump devices 1, for example those medical pump devices 1 which perform homecare treatment for the patients of the particular healthcare professional. The medical pump devices 1, in the view of Fig. 4, are identified by their serial number shown in the column indicated by reference number 700. For each medical pump device 1 herein different information in different fields 701-704 may be displayed or tasks may be performed. For example, in field 701 a status of a particular medical pump device 1 may be displayed, such as "delivery operation paused", "delivery operation currently in progress" or "device switched off", indicated in plain text or by specific symbols. In further fields it may be stated what the daily volume delivered with that medical pump device 1 is and what the daily prescription is. In field 702 it is displayed whether recent delivery operations of the medical pump device 1 conform to the actual prescription (so-called compliance information). Via field 703 a delivery profile may be accessed, and field 704 may be clicked to enter a new prescription or to modify an already existing prescription for a specific delivery pump device 1.

The noted information is displayed for all medical pump devices 1 associated with the web application account of the particular healthcare professional, and for all medical pump devices 1 the healthcare professional may for example enter or modify a prescription. Via the view 70 shown in Fig. 4, the healthcare professional hence may gain an overview over medical pump device 1 associated with his/her patients in an easy and comfortable manner.

The web application may also be accessible by relatives of a patient such that also relatives may monitor medical pump devices 1 of a particular related patient.

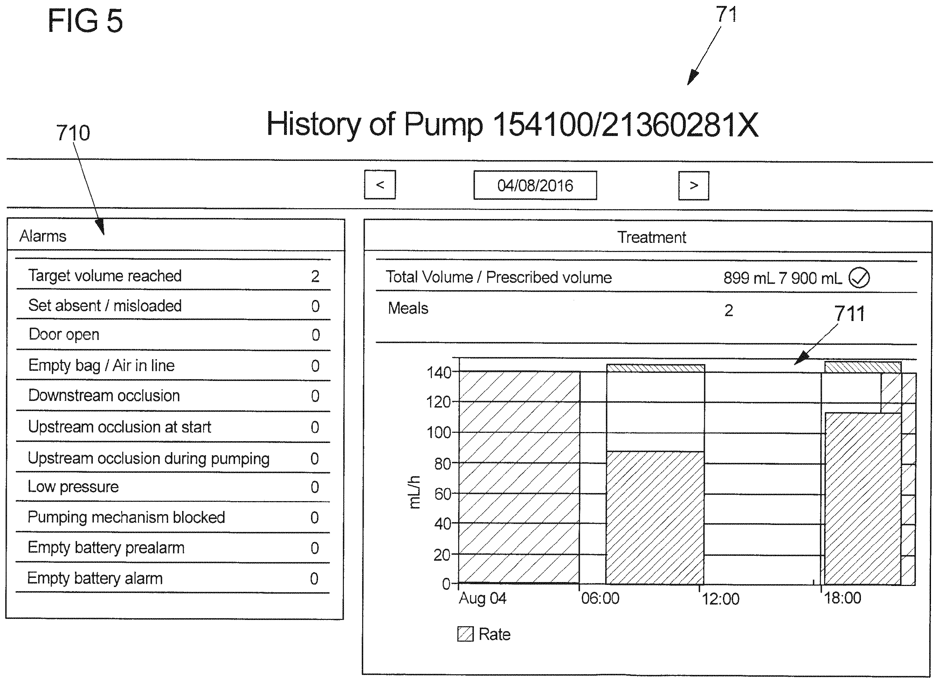

In another view 71, shown in Fig. 5, information relating to the history of usage of a medical pump device 1 may be displayed. In field 710, alarm conditions are displayed for a particular date. And in field 711 information relating to infusion operations carried out on that date are displayed over time.

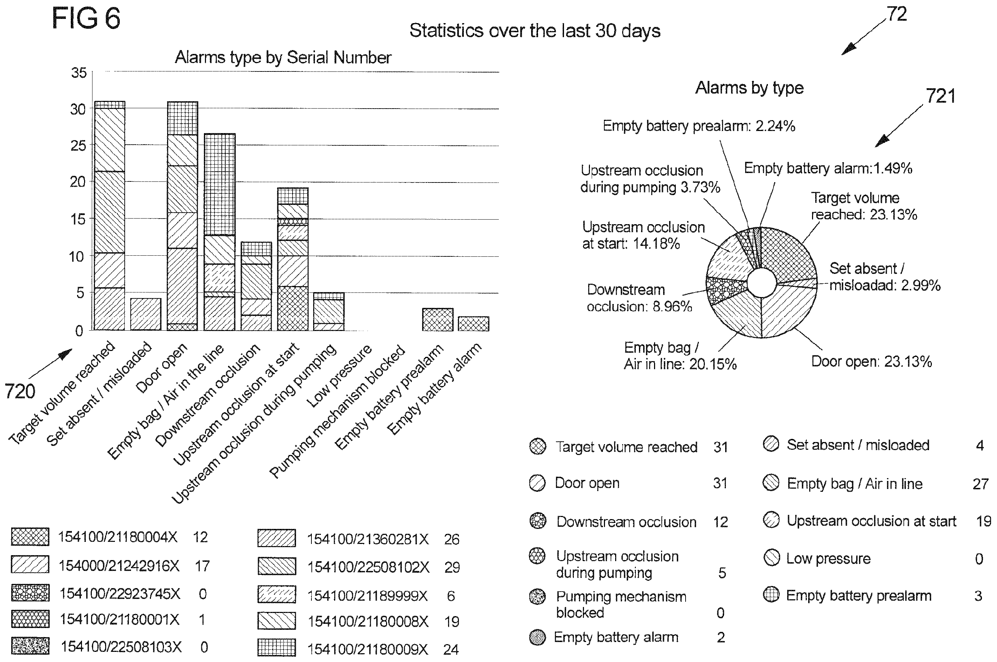

In another view 72 shown in Fig. 6 statistical information about a medical pump device 1 is displayed. In field 720, for example, a graph showing alarm conditions for the different medical pump devices 1 associated with the user are displayed. And in field 721 the occurrence percentage of alarms by type is displayed.

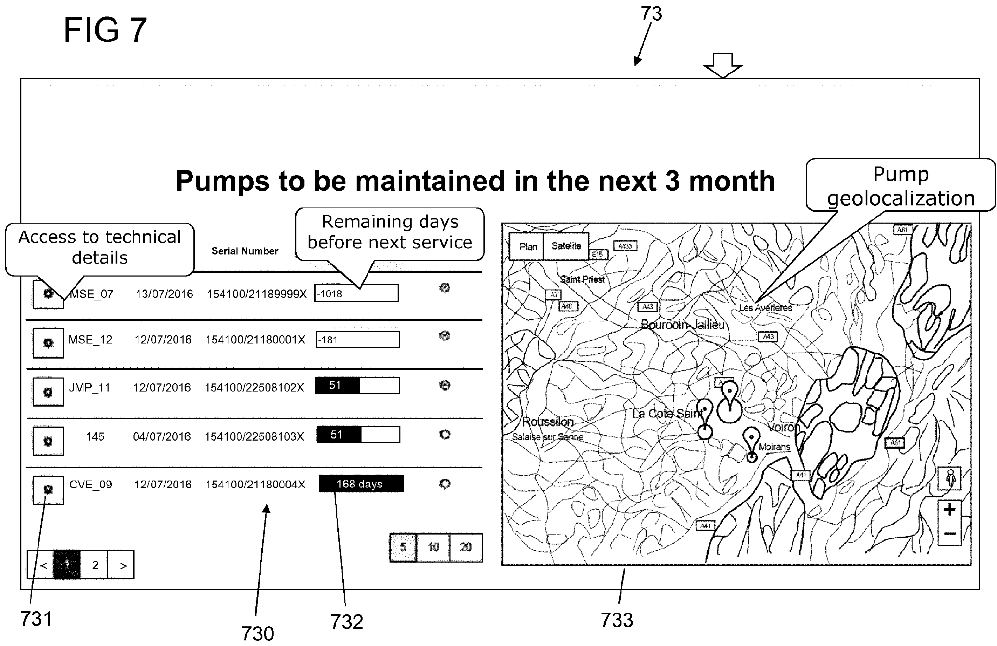

In another view 73 shown in Fig. 7 localization information may be displayed for different medical pump devices 1. In field 730 information relating to different medical pump devices 1 is displayed, wherein by clicking on field 731 technical details of a particular medical pump device 1 may be accessed. In field 732 it may be displayed how many days are left until a next maintenance service is required. In the geographical map of field 733 it is displayed where the medical pump devices 1 are located such that an overview about the geographical localization of the installed base of medical pump devices 1 is obtained.

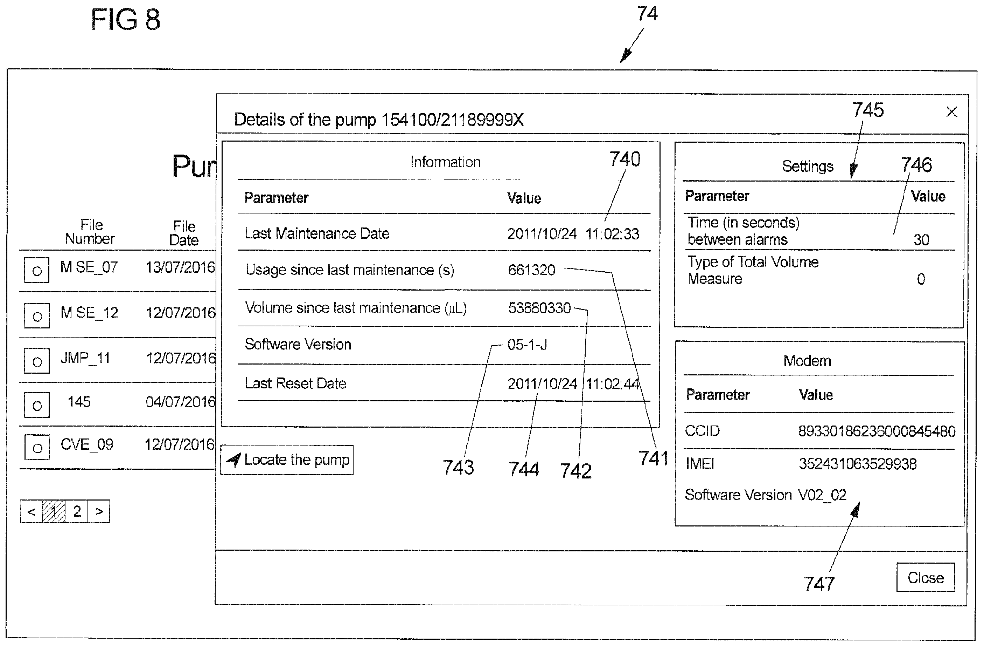

In yet another view 74 shown in Fig. 8 technical details with regard to a particular medical pump device 1 are displayed, as it is obtained by clicking on the field 731 in the view 73 of Fig. 7. Among the detailed technical information it is displayed when the last maintenance took place (field 740), the overall usage (in seconds) since the last maintenance (field 741), the overall delivered volume (in microliter) since the last maintenance (field 742), the current software version (field 743), and the date at which the medical pump device 1 has last been reset (field 744). In field 745 settings may be displayed, for example the time in between two alarms (field 746). In field 747 information relating to the modem of the medical pump device 1 is displayed.

The web application 7 may be used by healthcare professionals, such as physicians or nurses, and also by relatives of a patient to monitor and supervise patients. Herein, via the web application 7 a healthcare professional or a relative may obtain information relating to current and past delivery operations and hence may be able to determine whether the patient, which performs the delivery operations autonomously at for example his/her home 10, acts according to the prescription that the patient has obtained from the healthcare professional.

Via the web application 7, in addition, maintenance personnel may obtain information about a required maintenance of a medical pump device 1. In this way, the servicing of medical pump devices 1 may be facilitated.

Furthermore, the manufacturer may via the web application 7 obtain information relating to the usage of medical pump devices 1, information relating to preferred settings, and information relating to technical malfunctions of medical pump devices 1. This may help to continuously improve the medical pump devices 1.

The web application 7 may also allow for an export of data, for example as XML data such that the exported data is compatible with third-party devices.

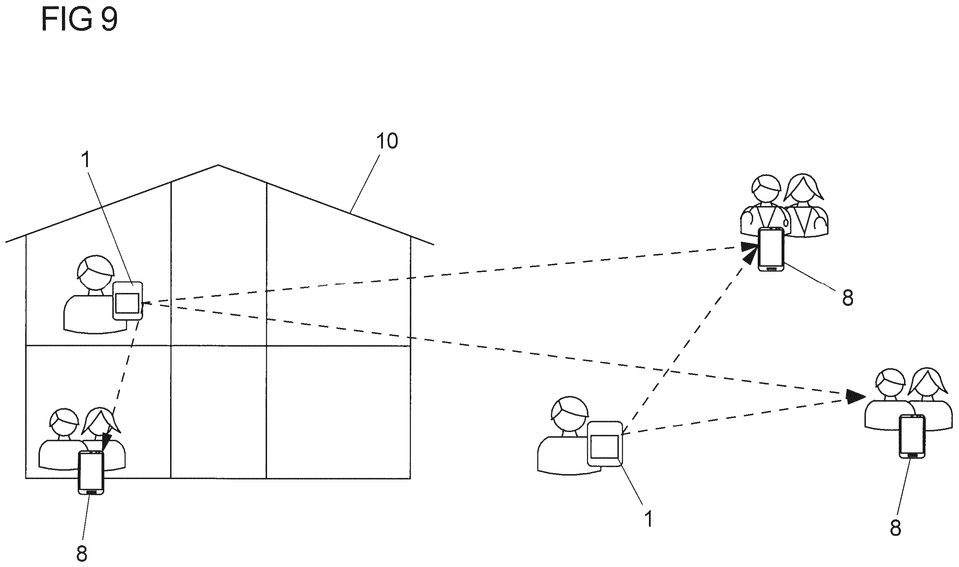

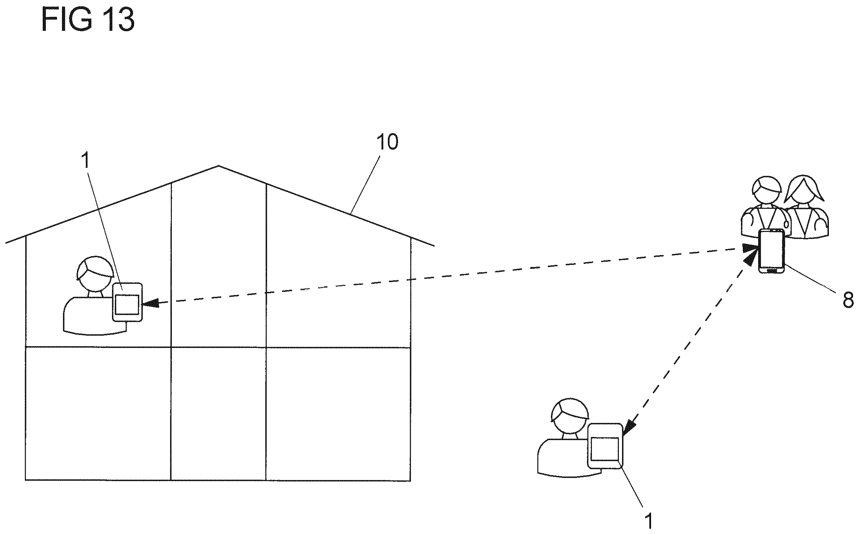

Fig. 9 to 14 display different possible use cases of the system as it is illustrated schematically in Fig. 1.

In a first use case illustrated in Fig. 9, a patient using a medical pump device 1 may be monitored by patient relatives or by a healthcare professional by accessing the web application 7 via a communication device 8 such as a smart phone or a tablet computer. For example, if a child is nourished by a medical pump device 1 at night, a parent having a sleeping room distant from the child's bedroom may be informed about an alarm condition via the web application 7.

In another example of this use case, a patient may be nourished by a medical pump device 1, and a patient relative may live not far from the patient. Via the web application 7 the patient relative may be informed in case a problem with the medical pump device 1 occurs or the medical pump device 1 is incorrectly used (for example when programming an excessive flowrate).

In another example of this use case, a patient may be nourished by pump, and a healthcare professional responsible for the patient may be located far away from the patient's home 10, for example 30 km away from the patient's home 10. Via the web application 7 the healthcare professional may be informed in case of a problem with the medical pump device 1 or an incorrect use of the medical pump device 1. Via the web application 7, in this case, the healthcare professional also receives detailed information about what the issue may be and hence may be enabled to support the patient remotely, for example by programming the medical pump device 1 remotely or by contacting the patient by telephone. In case of a severe problem the healthcare professional may also decide to visit the patient at home.

In case of alarm conditions additional messages may be effected by the web application 7, for example SMS messages or the like to communication devices 8 of a patient's relative or a healthcare professional.

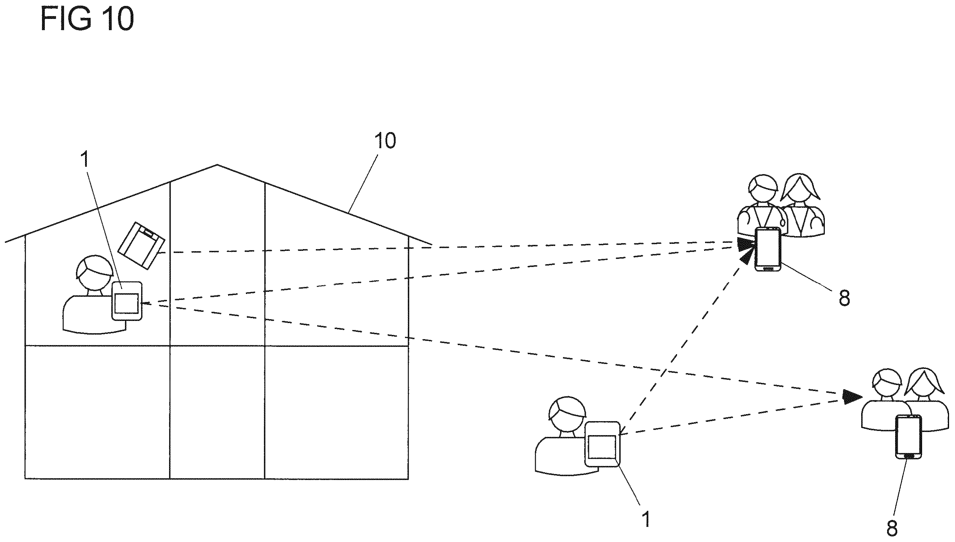

In another use case, illustrated in Fig. 10, a patient for example having a chronic disease may have to be enterally fed over an extended period of time. The patient may live far away from a responsible healthcare professional, for example 30 km away. In this case, via the web application 7 the healthcare professional can check on a daily basis if the patient follows the prescription. Also, the healthcare professional can trace the treatment of the patient and may transfer data relating to the treatment into an electronic medical record system. And the healthcare professional can check on the treatment's success and can potentially adapt the prescription.

In another example of this use case, a patient having a chronic disease implying a long-term enteral feeding by a medical pump device 1 may be willing to track the treatment as well as measure the outcome with regard to tangible indicators. If the patient for example has several connected health devices, the patient can gain an overview over treatment parameters using the web application 7. Furthermore, if the patient has a certain autonomy in term of the treatment administration, the patient may be able to determine an optimal compromise between treatment efficacy, comfort and way of life.

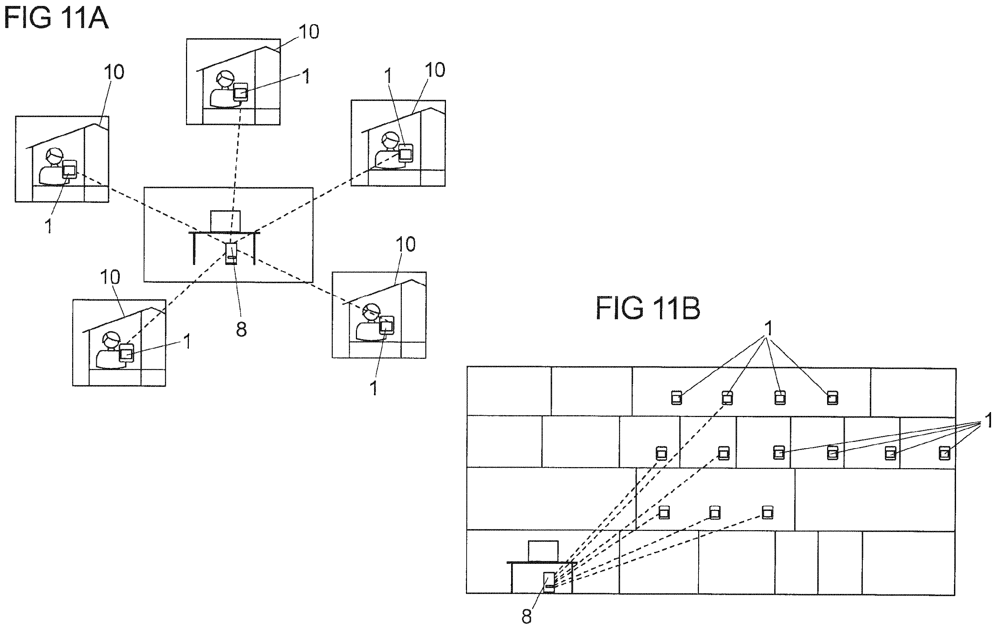

In another use case shown in Fig. 11A, a homecare service provider may be responsible for a number of medical pump devices 1, spread out over a substantial geographical territory. By means of the web application 7, the homecare service provider may perform services on the medical pump devices 1 at regular intervals, potentially dependent on the use of the devices, and may also attend to solving technical problems for example in case of alarms. In addition, the homecare service provider obtains an easy overview over the installed base, for example including the pump version, running time, the next maintenance date, key performance indicators relating to the reliability and use, location and the like.

In another use case shown in Fig. 11B, technical staff within a hospital may remotely manage medical pump devices 1 distributed throughout the hospital, may perform services on the medical devices 1 and may get an overview of the installed medical pump devices 1. In a hospital environment, by making use of the system proposed herein a hospital communication network is not affected, but communication between the medical pump devices 1 and communication devices 8 of technical hospital staff takes place independently from the hospital communication network (for example a WiFi network) making use of the low power wide area network.

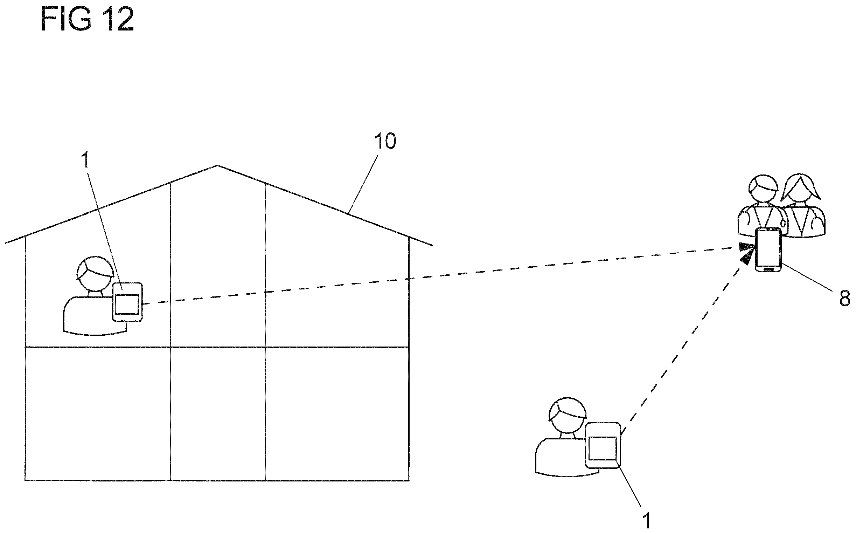

In another use case illustrated in Fig. 12, a patient may be supposed to receive a certain quantity of pharmaceutical solutions and device disposables in order to cover a given treatment period. For example, for the enteral feeding one pumpset a day may be needed. Via the web application 7, the healthcare professional in this case can check consumption from a remote location to potentially adapt and optimize a delivery plan for delivery of pharmaceutical solutions and device disposables. Also, the healthcare professional may be informed in case an abnormal consumption, for example an excessive pumpset consumption, occurs and may adapt and optimize the delivery plan accordingly.

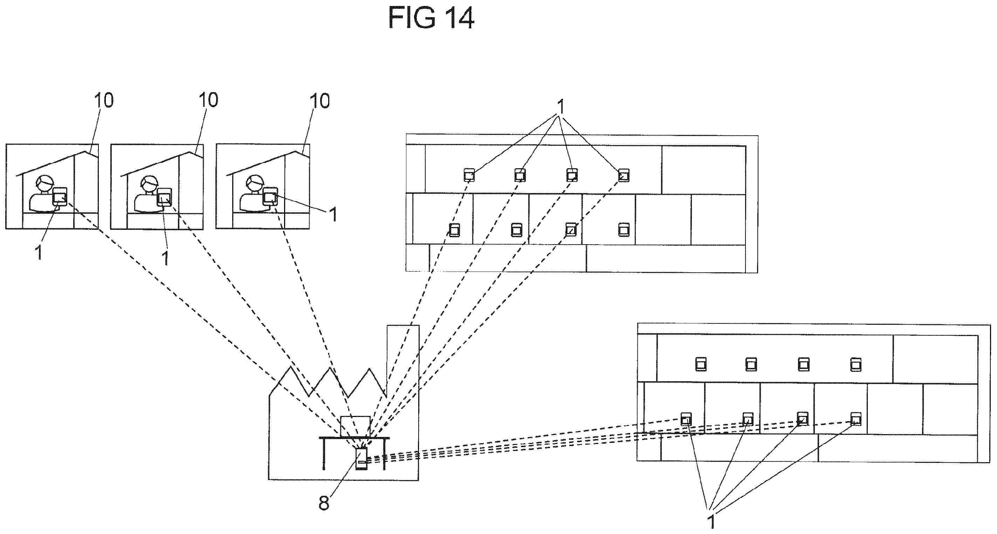

In yet another use case illustrated in Fig. 14, a device manufacturer may access the web application 7 to gain information about the usage of medical pump devices 1 and their reliability. Based on this information, continuous improvement of the medical pump devices 1 may be facilitated. For example, an overview of a typical use of medical pump devices 1 as well as key performance indicators with respect to product reliability may be obtained (for example statistics on functional alarms, technical alarms and the like). Based on these statistics technical issues may be detected, allowing for a preventive or corrective action.

In another example, the manufacturer may for example gain an overview of a software upgrade and its completion on the installed base of medical pump devices 1, for example in case of a bug fix in the software.

### List of Reference Numerals

- 1: Medical pump device
- 10: Patient's home
- 11: Communication module (LoRa module)
- 110: Antenna
- 12: Communication module (Bluetooth module)
- 120: Antenna
- 13: Communication module (GPS)
- 130: Antenna
- 2: External devices
- 3: gateway (base station)
- 4: Server
- 5: Wireless connection
- 6: Network connection
- 7: Web application
- 70: View
- 700-704: Fields
- 71: View
- 710, 711: Fields
- 72: View
- 720, 721: Fields
- 73: View
- 730-733: Fields
- 74: View
- 740-747: Fields
- 8: Communication device

## Claims

1. System for communicating information between a multiplicity of medical pump devices (1) and at least one communication device (8), the system comprising:
- a server (4) to which at least one communication device (8) is connectable,
- at least one gateway (3) connected to the server (4) via a network connection (6), and
- at least one medical pump device (1),
**characterized in that** the at least one medical pump device (1) is connected to the at least one gateway (3) via a low power wide area network connection (5), wherein the at least one medical pump device (1) comprises a first communication module (11) for establishing the low power wide area network connection (5) to the at least one gateway (3),
wherein the at least one medical pump device (1) comprises a second communication module (12) for establishing a communication connection according to a different network technology to another device (2, 8), such that a direct connection to the medical pump device (1) may be established, independent of the gateway.

2. System according to claim 1, **characterized in that** the low power wide area connection is a LoRaWAN connection.

3. System according to claim 1 or 2, **characterized in that** the communication connection is a Bluetooth connection, in particular a Bluetooth Low Energy connection.

4. System according to one of the preceding claims, **characterized in that** the at least one medical pump device (1) comprises a third communication module (12) for generating localization information.

5. System according to one of the preceding claims, **characterized in that** the at least one medical pump device (1) is located at a remote location from the at least one communication device (8).

6. System according to one of the preceding claims, **characterized in that** the server (4) is accessible via a web application (7) using a communication device (8) such as a computer device or a mobile communication device.

7. System according to claim 6, **characterized in that** the web application (7) is constituted to provide information relating to
- delivery operations carried out by the at least one medical pump device (1),
- alarm conditions of the at least one medical pump device (1),
- treatment compliance information of delivery operations carried out by the at least one medical pump device (1),
- patient information,
- maintenance information of the at least one medical pump device (1),
- pump settings and/or
- localization information of the at least one medical pump device (1).

8. Method for communicating information between a multiplicity of medical pump devices (1) and at least one communication device (8), in which:
- a server (4) communicates with at least one communication device (8), and
- at least one medical pump device (1) communicates with at least one gateway (3) connected to the server (4) via a network connection (6),
**characterized in that** the at least one medical pump device (1) communicates with the at least one gateway (3) via a low power wide area network connection (5), wherein the at least one medical pump device (1) comprises a first communication module (11) for establishing the low power wide area network connection (5) to the at least one gateway (3),
wherein the at least one medical pump device (1) communicates to another device (2, 8) with a second communication module (12) for establishing a communication connection according to a different network technology, such that a direct connection to the medical pump device (1) may be established, independent of the gateway.

## Patentansprüche

1. System zur Übermittlung von Informationen zwischen einer Vielzahl von medizinischen Pumpvorrichtungen (1) und mindestens einer Kommunikationsvorrichtung (8), wobei das System Folgendes umfasst:
- einen Server (4), an den mindestens eine Kommunikationsvorrichtung (8) angeschlossen werden kann,
- mindestens ein Gateway (3), das über eine Netzwerkverbindung (6) mit dem Server (4) verbunden ist, und
- mindestens eine medizinische Pumpvorrichtung (1),
**dadurch gekennzeichnet, dass** die mindestens eine medizinische Pumpvorrichtung (1) mit dem mindestens einen Gateway (3) über eine Low-Power-Wide-Area-Netzwerkverbindung (5) verbunden ist, wobei die mindestens eine medizinische Pumpvorrichtung (1) ein erstes Kommunikationsmodul (11) zum Herstellen der Low-Power-Wide-Area-Netzwerkverbindung (5) zu dem mindestens einen Gateway (3) umfasst,
wobei die mindestens eine medizinische Pumpvorrichtung (1) ein zweites Kommunikationsmodul (12) zum Herstellen einer Kommunikationsverbindung gemäß einer anderen Netzwerktechnologie zu einer anderen Vorrichtung (2, 8) umfasst, sodass unabhängig von dem Gateway eine direkte Verbindung zu der medizinischen Pumpvorrichtung (1) hergestellt werden kann.

2. System nach Anspruch 1, **dadurch gekennzeichnet, dass** die Low-Power-Wide-Area-Verbindung eine LoRaWAN-Verbindung ist.

3. System nach Anspruch 1 oder 2, **dadurch gekennzeichnet, dass** die Kommunikationsverbindung eine Bluetooth-Verbindung, insbesondere eine Bluetooth-Low-Energy-Verbindung ist.

4. System nach einem der vorhergehenden Ansprüche, **dadurch gekennzeichnet, dass** die mindestens eine medizinische Pumpvorrichtung (1) ein drittes Kommunikationsmodul (12) zum Erzeugen von Lokalisierungsinformationen umfasst.

5. System nach einem der vorhergehenden Ansprüche, **dadurch gekennzeichnet, dass** sich die mindestens eine medizinische Pumpvorrichtung (1) an einem von der mindestens einen Kommunikationsvorrichtung (8) entfernten Ort befindet.

6. System nach einem der vorhergehenden Ansprüche, **dadurch gekennzeichnet, dass** der Server (4) über eine Webanwendung (7) mit einer Kommunikationsvorrichtung (8) wie zum Beispiel einem Computergerät oder einem mobilen Kommunikationsgerät zugänglich ist.

7. System nach Anspruch 6, **dadurch gekennzeichnet, dass** die Webanwendung (7) so ausgebildet ist, dass sie Informationen bereitstellt über
- Abgabevorgänge, die von der mindestens einen medizinischen Pumpvorrichtung (1) durchgeführt werden,
- Alarmzustände der mindestens einen medizinischen Pumpvorrichtung (1),
- Informationen über die Einhaltung der Behandlungsvorschriften bei Abgabevorgängen, die von der mindestens einen medizinischen Pumpvorrichtung (1) durchgeführt wurden,
- Patienteninformationen,
- Wartungsinformationen über die mindestens eine medizinische Pumpvorrichtung (1),
- Pumpeneinstellungen und/oder
- Lokalisierungsinformationen der mindestens einen medizinischen Pumpvorrichtung (1).

8. Verfahren zur Übermittlung von Informationen zwischen einer Vielzahl von medizinischen Pumpvorrichtungen (1) und mindestens einer Kommunikationsvorrichtung (8), bei dem:
- ein Server (4) mit mindestens einer Kommunikationsvorrichtung (8) kommuniziert, und
- mindestens eine medizinische Pumpvorrichtung (1) mit mindestens einem Gateway (3) kommuniziert, das mit dem Server (4) über eine Netzwerkverbindung (6) verbunden ist,
**dadurch gekennzeichnet, dass** die mindestens eine medizinische Pumpvorrichtung (1) mit dem mindestens einen Gateway (3) über eine Low-Power-Wide-Area-Netzwerkverbindung (5) kommuniziert, wobei die mindestens eine medizinische Pumpvorrichtung (1) ein erstes Kommunikationsmodul (11) zum Herstellen der Low-Power-Wide-Area-Netzwerkverbindung (5) zu dem mindestens einen Gateway (3) umfasst,
wobei die mindestens eine medizinische Pumpvorrichtung (1) mit einer anderen Vorrichtung (2, 8) mit einem zweiten Kommunikationsmodul (12) zum Herstellen einer Kommunikationsverbindung gemäß einer anderen Netzwerktechnologie kommuniziert, sodass eine direkte Verbindung zu der medizinischen Pumpvorrichtung (1) hergestellt werden kann, unabhängig von dem Gateway.

## Revendications

1. Système de communication d'informations entre une multiplicité de dispositifs de pompe médicale (1) et au moins un dispositif de communication (8), le système comprenant :
- un serveur (4) auquel au moins un dispositif de communication (8) peut être connecté,
- au moins une passerelle (3) connectée au serveur (4) par une connexion réseau (6), et
- au moins un dispositif de pompe médicale (1),
**caractérisé en ce que** l'au moins un dispositif de pompe médicale (1) est connecté à l'au moins une passerelle (3) via une connexion réseau étendu à faible puissance (5), dans lequel l'au moins un dispositif de pompe médicale (1) comprend un premier module de communication (11) pour établir la connexion réseau étendu à faible puissance (5) à l'au moins une passerelle (3),
dans lequel l'au moins un dispositif de pompe médicale (1) comprend un deuxième module de communication (12) pour établir une connexion de communication à un autre dispositif (2, 8) selon une technologie de réseau différente, de telle sorte qu'une connexion directe au dispositif de pompe médicale (1) peut être établie, indépendamment de la passerelle.

2. Système selon la revendication 1, **caractérisé en ce que** la connexion étendue à faible puissance est une connexion LoRaWAN.

3. Système selon la revendication 1 ou 2, **caractérisé en ce que** la connexion de communication est une connexion Bluetooth, en particulier une connexion Bluetooth Low Energy.

4. Système selon l'une quelconque des revendications précédentes, **caractérisé en ce que** l'au moins un dispositif de pompe médicale (1) comprend un troisième module de communication (12) pour générer des informations de localisation.

5. Système selon l'une quelconque des revendications précédentes, **caractérisé en ce que** l'au moins un dispositif de pompe médicale (1) est situé à un endroit éloigné de l'au moins un dispositif de communication (8).

6. Système selon l'une quelconque des revendications précédentes, **caractérisé en ce que** le serveur (4) est accessible via une application web (7) en utilisant un dispositif de communication (8) tel qu'un dispositif informatique ou un dispositif de communication mobile.

7. Système selon la revendication 6, **caractérisé en ce que** l'application web (7) est constituée pour fournir des informations relatives à
- des opérations d'administration effectuées par l'au moins un dispositif de pompe médicale (1),
- des conditions d'alarme de l'au moins un dispositif de pompe médicale (1),
- des informations relatives à la conformité du traitement des opérations d'administration effectuées par l'au moins un dispositif de pompe médicale (1),
- des informations sur le patient,
- des informations de maintenance de l'au moins un dispositif de pompe médicale (1),
- des réglages de la pompe et/ou
- des informations de localisation de l'au moins un dispositif de pompe médicale (1).

8. Procédé de communication d'informations entre une multiplicité de dispositifs de pompe médicale (1) et au moins un dispositif de communication (8), dans lequel:
- un serveur (4) communique avec au moins un dispositif de communication (8), et
- au moins un dispositif de pompe médicale (1) communique avec au moins une passerelle (3) connectée au serveur (4) via une connexion réseau (6),
**caractérisé en ce que** l'au moins un dispositif de pompe médicale (1) communique avec l'au moins une passerelle (3) via une connexion réseau étendu à faible puissance (5), dans lequel l'au moins un dispositif de pompe médicale (1) comprend un premier module de communication (11) pour établir la connexion réseau étendu à faible puissance (5) à l'au moins une passerelle (3),
dans lequel l'au moins un dispositif de pompe médicale (1) communique avec un autre dispositif (2, 8) à l'aide d'un deuxième module de communication (12) pour établir une connexion de communication selon une technologie réseau différente, de telle sorte qu'une connexion directe au dispositif de pompe médicale (1) peut être établie, indépendamment de la passerelle.
